# EUROPEAN PATENT APPLICATION

(11) **EP 0 537 393 A1**
(43) Date of publication of application: **21.04.1993**
(21) Application number: 91309564.2
(22) Date of filing: 17.10.1991
(51) Int. Cl.: A61L 9/12

(54) **Vibrating diffusion-type aromatic device**

(71) Applicant: Yang, Tai-Her, Taipei (TW)
(72) Inventor: Yang, Tai-Her, Taipei (TW)
(74) Representative: Arthur, Bryan Edward

(57) **Abstract**

The present invention relates to mount the aromatic on the magnetic reed and use a coil to attract the reed in order to spread aroma and in substitute for the traditional way that uses fan motor to spread aroma. The vibrating diffusion type aromatic device according to the invention is effective to reduce the production cost and save the dimensions and weight.

## Description

### BACKGROUND OF THE INVENTION

The conventional diffusion type aromatic device usually uses a rotary motor to drive the fan which provides air flow spreading aromatic into the air for improving air quality. As the fan being a motion type element needs a motor and rotary blades, the cost is higher and dimensions greater. The present invention is relates a device that has an electric magnet provided attraction and rebound to shake the aromatic on the reed for spreading the aroma into the air.

### SUMMARY OF THE INVENTION

The present invention relates to mount the aromatic on the magnetic reed and use a coil to attract the reed in order to spread aroma and in substitute for the traditional way that uses fan motor to spread aroma. The vibrating diffusion type aromatic device according to the present invention is effective to reduce the production cost and save the dimensions and weight.

### BRIEF DECRIPTION OF THE DRAWINGS

FIG. 1 is a structural diagram showing the vibrating diffusion type aromatic device according to the present invention.

FIG. 2 is a diagram showing the first vibrating operation circuit in FIG. 1.

FIG. 3 is a diagram showing the second vibrating operation circuit in FIG. 1.

### DETAILED DESCRIPTION OF THE INVENTION

Aromatic device in general has been widely applied in the car, office, private room, bathroom to create an aromatic environment. However, most of such devices concern a motor for driving the fan and spreading aroma so that production cannot be accomplished at a reasonable cost and dimensions. The present invention is to provide a vibrating diffusion type aromatic device comprising power supply, vibrating unit, aromatic and housing. As it has a small dimensions, it can be made into a portable or desk top or hanging type in convenience for use.

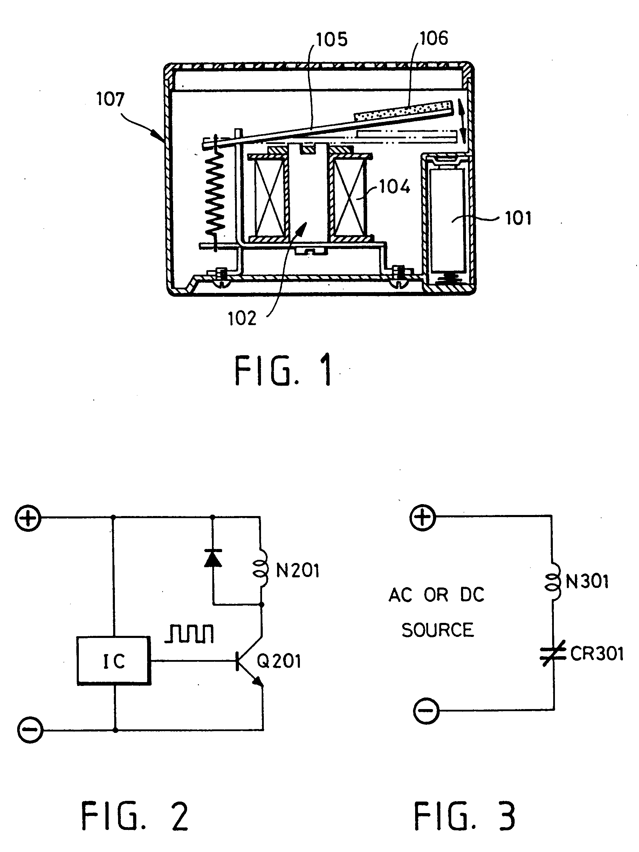

The structure of said device according to the present invention is described in details as below:
Referring to FIG. 1, power supply 101 including battery assembly or AC or rectified DC, magnetic device 10 including coil 104 for attracting the reed 105, and magnetic reed 105 being provided to mount aromatic 106 being spreading under vibration, vibrating operation circuit provided to drive the coil, and housing 107. Upon power transmission, magnetic reed may be vibrated continuously (super low frequency below 20 HZ in general) to spread aroma into the air.

Referring to FIG. 2, the circuit is operated by periodic pulse, wherein periodic pulse IC generates a periodic ON, OFF signal to drive SW transistor Q201 and further enable the coil N201 with power for attracting the magnetic reed to vibrate relatively in order to spread aromatic mounted on the reed.

Referring to FIG. 3, another embodiment of circuit is operated by a frequent closed contact assembly CR 301 comprising conductive reeds, breaking during attraction, and during the rebound of reeds closed contact assembly driving the coil N301 in free vibration to spread aromatic on the reeds.

To conclude the descriptions mentioned above, the vibrating diffusion type aromatic device can mount the aromatic on the reeds and use the coil to attract the reeds and further spread aroma in substitute for the conventional spreading type by fan motor. Therefore, the production can be accomplished at reasonable cost and dimensions. The present invention has conformed to the requirements of new utility model. Your examination in accordance with the law will be highly appreciated.

## Claims

1. A vibrating diffusion type aromatic device comprising power supply, vibrating unit, aromatic and housing, power supply including battery assembly or AC or rectified DC, magnetic device including coil for attracting the reed, and magnetic reed being provided to mount aromatic spreading under vibration, vibrating operation circuit provided to drive the coil, when power supply to magnetic reed, it being vibrated continuously (super low frequency below 20 HZ in general) to spread aroma into the air.

2. The vibrating diffusion type aromatic device according to claim 1 wherein the circuit being operated by periodic pulse, and periodic pulse IC generating a periodic ON, OFF signal to drive SW transistor and further enabling the coil with power for attracting the magnetic reed to vibrate relatively in order to spread aromatic mounted on the reed.

3. The vibrating diffusion type aromatic device according to claim 1 wherein the vibrating operation circuit may further be operated by a frequent closed contact assembly comprising conductive reeds, breaking during attraction, and during the rebound of reeds closed contact assembly driving the coil in free vibration to spread aromatic on the reeds.
